# EUROPEAN PATENT SPECIFICATION

(11) **EP 2 589 376 B1**
(45) Date of publication and mention of the grant of the patent: **21.09.2016**
(21) Application number: 12151435.0
(22) Date of filing: 17.01.2012
(51) Int. Cl.: A61K 9/00, A61K 9/20, A61K 31/445

(54) **Oral disintegrating composition of anti-histamine agents**
Im Mund zerfallende Zusammensetzung aus Anti-Histamin-Wirkstoffen
Composition à désintégration orale d'agents anti-histamine

(30) Priority: 01.11.2011 IN CH37342011
(43) Date of publication of application: 08.05.2013
(73) Proprietor: Inopharm Limited, 1304 Nicosia (CY)
(72) Inventor: Raju, N S V, 560035 BANGALORE (IN); Raghupathi, Kandarapu, HYDERABAD (IN); Rao Maram, Sambasiva, 522601 Narasaraopet (IN)
(74) Representative: Regimbeau

(56) References cited:
- EP-A1- 0 134 124
- WO-A2-2006/135689
- CN-A- 102 218 041
- US-A1- 2005 003 001
- US-A1- 2008 145 438
- US-A1- 2010 105 783

## Description

The present invention relates to oral disintegrating composition of anti-histamine agents. Particularly, the present invention relates to oral disintegrating composition of ebastine. More particularly, the present invention relates to non-lyophilized composition of ebastine and its process for preparing the same.

Ebastine is a long-acting and selective H1-histamine receptor antagonist. Chemically ebastine is 4-(4-benzhydryloxy-1-piperidyl)-1-(4-tert-butylphenyl)butan-1-one and its having following chemical structure:

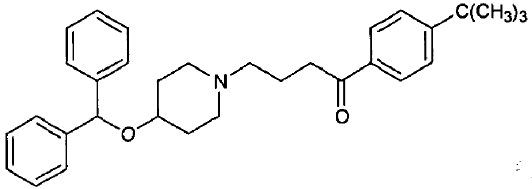

It is practically insoluble in water, very soluble in methylene chloride, sparingly soluble in methanol. It is used for the treatment of seasonal allergic rhinitis as well as symptomatic treatment of urticaria. It is available as immediate release tablets as well as orally disintegrating tablets in various markets. Immediate release tablets are available under the brand name of KESTINE® and in recent years orally disintegrating tablets were developed by Almirall, Spain using lyophilized technology. These orally disintegrating tables overcome the difficulties using common immediate release tablets like easy administration to geriatric patients, pediatric patients, those having swallowing problems; can be administered during travelling without necessity of water etc. The following patents disclose various formulation technologies of orally disintegrating tablets:
EP134124 patent discloses and claims ebastine and its preparation.

EP614362 patent discloses micronized ebastine. Due to its low solubility, this patent discloses use of micronized Ebastine. This patent describes various formulation trails using micronized and un-micronized ebastine with direct compression and wet granulation technique and concluded that trial with micronized grade of ebastine using wet granulation improved the dissolution. This patent also discloses freeze drying process for the preparation of ebastine with an improved disintegration time of less than 45 seconds. The preferred micronized ebastine average particle size disclosed in this patent is less than 25 microns.

EP1552851 patent discloses intrabuccally rapidly disintegrating tablet containing D-mannitol, disintegrant, and 0.01 % to 0.5 % by weight of stearic acid or a metallic stearate, where the average particle size of D-mannitol is 31-80 microns. This patent also describes the manufacturing process of intrabuccally rapidly disintegrating tablet, where the tablets are compressed using "external lubricating compression method" technology which involves spraying lubricant stearic acid or a metallic stearate on to the punches and without adding lubricant to the blend.

EP1716848 patent discloses solid pharmaceutical composition containing ebastine and non-ionic tensioactive agent. The non-ionic tensioactive agents disclosed in this patent are selected from among the group consisting of fatty esters of polyethoxylenated sorbitan, marketed under the name of POLYSORBATE 80. This patent discloses various examples of compositions with non-ionic tensioactive agents (Polysorbate) and anionic or amphoteric tensioactive like Sodium laurylsulphate, Lecithin, Sodium docusate and showed that the composition with non-ionic tensioactive agents gave good dissolution compared to anionic or amphoteric tensioactive agents.

EP1898882 patent discloses nanoparticulate composition of ebastine containing average particle size of less than about 2000nm and also the composition further contains one or more surface stabilizers.
EP 1944028 patent discloses composition in the form of matrix containing ebastine dispersed in nonionic surfactant. The preferred nonionic surfactant should have HLB value of between 10 and 20 and also melting point 30°C and 70°C.

CN 102218041 discloses solid dispersions comprising ebastine and poloxamer.

The above mentioned prior-art documents disclose various technologies for improving drug solubility as well as bioavailability like use of micronized ebastine, use of D-mannitol 31-80 microns, use of non-ionic tensioactive agents, nanoparticles and use of nonionic surfactant having HLB value of 10-20. Still there is need to develop simple process and composition for oral disintegrating tablets of ebastine. The inventors of the present invention developed simple composition and process of ebastine, comprising surfactant of HLB value more than 20 and one or more pharmaceutically acceptable excipients.

Accordingly, the present invention provides orally disintegrating composition of anti-histamine drug comprising surfactant of HLB (hydrophilic-lipophilic balance) value more than 20 and one or more pharmaceutically acceptable excipients, wherein the anti-histamine drug is ebastine, wherein surfactant of HLB value more than 20 is selected from the group consisting of Poloxamer, in particular PEG-PPG-PEG Poloxamer 188, PEG-PPG-PEG Poloxamer 338 , PEG-PPG-PEG Poloxamer 237, and combinations thereof, and wherein surfactant of HLB value more than 20 is present in an amount of less than 5 %, by weight compared to the total weight of the composition.

Orally disintegrating tablets (ODTs) have gained much attention as a preferred alternative to conventional oral dosage forms such as tablets and capsules. An ODT is a solid dosage form that disintegrates and dissolves in the mouth (either on or beneath the tongue or in the buccal cavity) without water within 60 seconds or less. These tablets are distinguished from conventional sublingual tablets, lozenges, and buccal tablets which require more than a minute to dissolve in the mouth. In the literature, ODTs also are called orally disintegrating, orodisperse, mouth-dissolving, quick-dissolve, fast-melt, and rapid-disintegrating tablets and freeze-dried wafers.

Anti-histamine drug according to the present invention is ebastine pharmaceutically acceptable salts thereof.

The phrase "pharmaceutically acceptable salt(s)", as used herein, means those salts of anti-histamine drugs that are safe and effective for use in mammals and that possess the desired biological activity. Suitable pharmacologically acceptable salts of antihistamines are in particular water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)-benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methane-sulfonic acid or 1-hydroxy-2-naphthoic acid, the acids being employed in salt preparation--depending on whether it is a mono- or polybasic acid and depending on which salt is desiredin an equimolar quantitative ratio or one differing therefrom.

Anti-histamine drug, in particular ebastine, is not limited by the particle size and is preferably in a proportion by weight comprised between 2% and 10%, more preferably between 5% and 7%, compared to the total weight of the composition. The content in ebastine is preferably adapted to provide a dosage of 10 mg or 20mg of ebastin per tablet.

Anti-histamine drug, in particular ebastine, can be present in the composition in micronized form or in un-micronized form, preferably anti-histamine drug, in particular ebastine, is micronized.

In a preferred embodiment of the invention, the composition is non-lyophilized. The composition is advantageously under the form of tablets.

The selection of additional excipients represents routine work for the skilled person, taking into account his common general knowledge and the usual handbooks and reference books in pharmaceutical technology such as the Handbook of Pharmaceutical excipients, Sixth edition edited by Raymond C Rowe, Paul J Sheskey and Marian E Quinn.

The one or more pharmaceutically acceptable excipients are preferably selected from the group consisting of diluents, binders, disintegrants, hydrophilic agents, flavouring agents, sweetening agents, lubricants, glidants and combinations thereof.

Diluents are inactive excipients which are generally used to increase the bulk of the tablet, thus facilitating easy of tablet or capsule preparations. Suitable diluents used according to the present invention may be selected from microcrystalline cellulose, mannitol, lactose, starch, calcium hydrogen phosphate, sorbitol, sucrose, dicalcium phosphate and combinations thereof. Mannitol is preferred.

Binders are used in the formulation of solid oral dosage forms to hold the active pharmaceutical ingredient and inactive ingredients together in a cohesive mix. Suitable binders used according to the present invention may be selected from the group consisting of povidone, potato starch, wheat starch, corn starch, hydroxypropyl methylcellulose, hydroxypropyl cellulose, hydroxyethyl cellulose, gelatin and combinations thereof. Hydroxypropyl methylcellulose is preferred.

Disintegrants are excipients causing a quick breaking of the tablet in contact of aqueous medium and also a quick disintegration of the granules, such that the anti-histamine drug is quickly released. Suitable disintegrants used according to the present invention may be selected from the group consisting of sodium starch glycolate, crospovidone, microcrystalline cellulose, low substituted hydroxypropyl cellulose, croscarmellose sodium, carmellose calcium, croscarmellose potassium, silicified microcrystalline cellulose and combinations thereof. Carmellose calcium, crospovidone, microcrystalline cellulose, croscamellose sodium, and combinations thereof are preferred.

Suitable hydrophilic agents used to the present invention may be selected from the group consisting of povidone, hydroxypropyl methylcellulose, hydroxypropyl cellulose, hydroxyethyl cellulose, gelatin and combinations thereof. Hydroxypropyl methylcellulose, povidone, gelatin, and combinations thereof are preferred.

Suitable flavouring agents used to the present invention may be selected from from the group consisting of trusil peppermint, vanilla, adipic acid, ethyl cellulose, limonene, isoamyl acetate and combinations thereof.

Suitable sweetening agents used to the present invention may be selected from the group consisting of neotame, aspartame, saccharin, sucralose, stevia, cyclamate, sucrose, mannitol, acesulfame potassium, alitame and combinations thereof.

Lubricants are excipients reducing tensions between particles, favoring the formation of the tablets and preventing the adhesion of the particles. Suitable lubricants used according to the present invention may be selected from the group consisting of sodium stearyl fumarate, magnesium stearate, calcium stearate, hydrogenated vegetable oil, stearic acid, glyceryl behenate, talc and the like, and combinations thereof. Magnesium stearate is preferred.

Glidants are used to promote powder flow by reducing interparticle friction and cohesion. Suitable glidants used according to the present invention may be selected from the group consisting of calcium phosphate tribasic, powdered cellulose, colloidal silicon dioxide, magnesium oxide, magnesium silicate, magnesium trisilicate, starch, talc and combinations thereof. Colloidal silicon dioxide is preferred.

Suitable surfactants of HLB more than 20 used according to the present invention may be selected from the group consisting of Poloxamer,in particular PEG-PPG-PEG Pluronic^{®} F-68 (synonym of Poloxamer 188), PEG-PPG-PEG Pluronic^{®} F-108 (synonym of Poloxamer 338), PEG-PPG-PEG Pluronic^{®} F-87 (synonym of Poloxamer 237), and combination thereof. Preferred surfactant is poloxamer 188. Poloxamer are polyoxyethylene-polyoxypropylene block copolymer nonionic surfactants. The polyoxyethylene segment is hydrophilic whereas the polyoxypropylene segment is hydrophobic. All poloxamer are chemically similar in composition, differing only in the relative percentage of propylene and ethylene blocks.

Poloxamer block copolymers are available from BASF Corporation under the Pluronic® registered tradename. The Pluronic® block copolymers are synthetic copolymers of ethylene oxide and propylene oxide represented by the following chemical structure:
HO(C₂H₄O)ₐ(C₃H₆O)_{b}(C₂H₄O)ₐH wherein a and b blocks have the following values:

**Table 1: poloxamer block copolymers**

| Pluronic® | Poloxamer | a | b | HLB |
|---|---|---|---|---|
| F68NF | 188 | 80 | 27 | >24 |
| F87NF | 237 | 64 | 37 | >24 |
| F108NF | 338 | 141 | 44 | >24 |

Suitable surfactants of the invention preferably have a HLB value less than 35, more preferably less than 30.

The amount of Poloxamer present in the composition is less than 5% by weight of poloxamer compared to the total weight of the composition. In a preferred embodiment, the composition contains at least 2% by weight of poloxamer compared to the total weight of the composition.

A preferred composition, according to the invention, comprises:
- Ebastine;
- Poloxamer, in particular poloxamer 188 (HO(C₂H₄O)ₐ(C₃H₆O)_{b}(C₂H₄O)ₐH wherein a is 80 and b is 27);
- at least one diluent, in particular mannitol;
- at least one binder, in particular hydroxypropyl methylcellulose;
- at least one disintegrant, in particular selected from the group consisting of carmellose calcium, crospovidone, microcrystalline cellulose, croscamellose sodium, and combinations thereof;
- at least one hydrophilic agent, in particular selected from the group consisting of hydroxypropyl methylcellulose, povidone, gelatin, and combinations thereof;
- at least one sweetening agent;
- at least one flavouring agent;
- at least one lubricant, in particular magnesium stearate;
- at least one glidant, in particular colloidal silicon dioxide;
- optionally water;
and is preferably under the tablet form.

The content in each ingredient is adapted by the skilled person on the basis of his common general knowledge and the usual handbooks and reference books in pharmaceutical technology. The tablet preferably contains 10 mg or 20mg of ebastin.

In another embodiment of the present invention provides a process for preparing orally disintegrating tables of anti-histamine drug comprising surfactant of HLB value more than 20 (and preferably less than 35).

Orally disintegrating tablets of the present invention may be prepared by wet granulation, dry granulation and direct compression process. Preferably orally disintegrating tablets according to the present invention is prepared by wet granulation process. These processes are well-known by the skilled person.

Suitable solvents used according to the present invention may be selected from water, ethanol, isopropyl alcohol, and combinations thereof.

Orally disintegrating tablets of ebastine according to the present invention may be used for the symptomatic treatment of seasonal allergic rhinitis and perennial and symptomatic treatment of urticaria.

The invention is illustrated by the following non limiting examples:

### Example 1: Composition of ebastine orally disintegrating tablets as per the present invention

### Example 2: Composition of ebastine orally disintegrating tablets without Poloxamer

**Table 2: examples of formulation (comparative and invention)**

| | **Example 1** | **Example 2** |
|---|---|---|
| **NAME OF INGREDIENTS** | **% w/w** | **% w/w** |
| EBASTINE | 6.6 | 6.6 |
| hydroxypropyl methylcellulose | 0.4 | 0.4 |
| Povidone | 0.5 | 0.5 |
| Poloxamer 188 | 2 | - |
| Gelatin | 1.5 | 1.5 |
| Carmellose calcium | 15.5 | 15.5 |
| Crospovidone | 8 | 8 |
| Mannitol | 50 | 52 |
| Microcrystalline cellulose | 10.55 | 10.55 |
| Croscarmellose sodium | 2 | 2 |
| colloidal silicon dioxide | 1.66 | 1.66 |
| Trusil peppermint | 0.67 | 0.67 |
| Neotame | 0.07 | 0.07 |
| Magnesium stearate | 0.55 | 0.55 |
| Purified water | qs | qs |
| **Total** | **100**.**0** | **100**.**0** |

### Process:

### Example 1:

**Sifting:** carmellose calcium, mannitol, crospovidone, croscarmellose sodium, colloidal hydrated silica, microcrystalline cellulose neotame, trusil peppermint and magnesium stearate were sifted separately through #40 mesh.

**Drug dispersion preparation:** Dissolve povidone, hypromellose and poloxamer 188 in purified water under continuous stirring till to get a clear solution. Add ebastine to this solution with continuous stirring to get a homogenous dispersion.

Purified water was heated up to 60°C and gelatin was added to dissolve. This gelatin solution was added to the above drug dispersion and mixed for 15 minutes to get homogenized drug dispersion.

**Granulation:** carmellose calcium was loaded in rapid mixer granulator and the drug dispersion solution was slowly added and granulated to get consistent granules. The granules were dried in fluid bed drier.

### Blending:

The above dried granules were blended with pre-sifted mannitol, croscarmellose sodium, crospovidone, colloidal hydrated silica, neotame, microcrystalline cellulose and trusil peppermint flavour in octagonal blender for 15 minutes.

Magnesium stearate was added to the above step and lubricated in octagonal blender for 3 minutes.

### Compression:

Compresse the lubricated blend using 11.0 mm flat faced bevelled edged circular shape punches.

**Example 2:** The process is same as that of above given for Example 1 except poloxamer addition.

Dissolution profile was conducted for both the tablets of Example 1 & 2 in pH 4.5 acetate buffer with 0,1% SLS medium, 900 ml using USP 2 apparatus at 50 rpm.

**Table 3: dissolution profile**

| **Time in minutes** | **Reference Product** | **Example 1** | **Example 2** |
|---|---|---|---|
| 10 | 83 | 86 | 65 |
| 15 | 87 | 89 | 68 |
| 30 | 89 | 90 | 70 |
| 45 | 89 | 93 | 71 |
| 60 | 90 | 92 | 73 |

The above dissolution data revealed that use of poloxamer enhanced the solubility of ebastine and also matching with reference product.

## Claims

1. Orally disintegrating composition of anti-histamine drug, wherein the composition comprises anti-histamine drug, surfactant of Hydrophilic Lipophilic Balance (HLB) value more than 20, and one or more pharmaceutically acceptable excipients,
wherein the anti-histamine drug is ebastine,
wherein surfactant of HLB value more than 20 is selected from the group consisting of Poloxamer, in particular PEG-PPG-PEG Poloxamer 188, PEG-PPG-PEG Poloxamer 338 , PEG-PPG-PEG Poloxamer 237, and combination thereof,
and wherein surfactant of HLB value more than 20 is present in an amount of less than 5 %, by weight compared to the total weight of the composition.

2. Orally disintegrating composition of claim 1, wherein surfactant of HLB value more than 20 is present in an amount of a least 2 %, by weight compared to the total weight of the composition.

3. Orally disintegrating composition of claim 1 or 2, wherein one or more pharmaceutically acceptable excipients are selected from the group consisting of diluents, binders, disintegrants, hydrophilic agents, flavouring agents, sweetening agents, lubricants, glidants and combinations thereof.

4. Orally disintegrating composition of any one of the preceding claims, wherein the anti-histamine drug, in particular ebastine, is micronized.

5. Orally disintegrating composition of any one of the preceding claims, wherein the composition is under the form of tablets.

6. Orally disintegrating composition of any one of the preceding claims, wherein the composition is prepared by direct compression, wet granulation and dry granulation, most preferably by wet granulation.

## Patentansprüche

1. Im Mund zerfallende Zusammensetzung eines Anti-Histamin-Wirkstoffs, wobei die Zusammensetzung einen Anti-Histamin-Wirkstoff, ein Tensid mit einem Hydrophile-Lipophile-Gleichgewichtswert (HLB-Wert) von mehr als 20 und einen oder mehrere pharmazeutisch akzeptable Hilfsstoffe umfasst,
wobei der Anti-Histamin-Wirkstoff Ebastin ist,
wobei das Tensid mit einem HLB-Wert von mehr als 20 aus der Gruppe bestehend aus Poloxamer, insbesondere PEG-PPG-PEG-Poloxamer 188, PEG-PPG-PEG-Poloxamer 338, PEG-PPG-PEG-Poloxamer 237 und Kombinationen davon ausgewählt ist
und wobei das Tensid mit einem HLB-Wert von mehr als 20 in einer Menge von weniger als 5 Gew.-% im Vergleich zu dem Gesamtgewicht der Zusammensetzung vorliegt.

2. Im Mund zerfallende Zusammensetzung nach Anspruch 1, wobei das Tensid mit einem HLB-Wert von mehr als 20 in einer Menge von mindestens 2 Gew.-% im Vergleich zu dem Gesamtgewicht der Zusammensetzung vorliegt.

3. Im Mund zerfallende Zusammensetzung nach Anspruch 1 oder 2, wobei der eine oder die mehreren pharmazeutisch akzeptablen Hilfsstoffe aus der Gruppe bestehend aus Verdünnungsmitteln, Bindemitteln, Sprengmitteln, hydrophilen Mitteln, Geschmacksstoffen, Süßungsmitteln, Gleitmitteln, Fließregulierungsmitteln und Kombinationen davon ausgewählt ist bzw. sind.

4. Im Mund zerfallende Zusammensetzung nach einem der vorhergehenden Ansprüche, wobei der Anti-Histamin-Wirkstoff, insbesondere Ebastin, mikronisiert ist.

5. Im Mund zerfallende Zusammensetzung nach einem der vorhergehenden Ansprüche, wobei die Zusammensetzung in der Form von Tabletten ist.

6. Im Mund zerfallende Zusammensetzung nach einem der vorhergehenden Ansprüche, wobei die Zusammensetzung durch Direkttablettierung, Feuchtgranulierung und Trockengranulierung, am meisten bevorzugt durch Feuchtgranulierung hergestellt wird.

## Revendications

1. Composition à désintégration orale de médicament antihistaminique, dans laquelle la composition comprend un médicament antihistaminique, un tensioactif dont la valeur d'équilibre hydrophile-lipophile (HLB) est supérieure à 20, et un ou plusieurs excipients pharmaceutiquement acceptables,
dans laquelle le médicament antihistaminique est l'ébastine,
dans laquelle le tensioactif dont la valeur de HLB est supérieure à 20 est sélectionné dans le groupe consistant en les poloxamères, en particulier le PEG-PPG-PEG poloxamère 188, le PEG-PPG-PEG poloxamère 338, le PEG-PPG-PEG poloxamère 237, et leurs combinaisons, et dans laquelle le tensioactif dont la valeur de HLB est supérieure à 20 est présent en une quantité inférieure à 5 %, en poids par rapport au poids total de la composition.

2. Composition à désintégration orale selon la revendication 1, dans laquelle le tensioactif dont la valeur de HLB est supérieure à 20 est présent en une quantité d'au moins 2 %, en poids par rapport au poids total de la composition.

3. Composition à désintégration orale selon la revendication 1 ou 2, dans laquelle un ou plusieurs excipients pharmaceutiquement acceptables sont sélectionnés dans le groupe consistant en les diluants, les liants, les désintégrants, les agents hydrophiles, les agents aromatisants, les agents édulcorants, les lubrifiants, les glissants et leurs combinaisons.

4. Composition à désintégration orale selon l'une quelconque des revendications précédentes, dans laquelle le médicament antihistaminique, en particulier l'ébastine, est micronisé.

5. Composition à désintégration orale selon l'une quelconque des revendications précédentes, dans laquelle la composition est sous la forme de comprimés.

6. Composition à désintégration orale selon l'une quelconque des revendications précédentes, dans laquelle la composition est préparée par compression directe, granulation par voie humide et granulation par voie sèche, de manière préférée entre toutes par granulation par voie humide.
